# EUROPÄISCHE PATENTSCHRIFT

(11) **EP 3 063 541 B1**
(45) Veröffentlichungstag und Bekanntmachung des Hinweises auf die Patenterteilung: **06.01.2021**
(21) Anmeldenummer: 14795998.5
(22) Anmeldetag: 28.10.2014
(51) Int. Cl.: G01N 33/49, A61B 5/1455, G01N 21/31

(54) **VERFAHREN UND VORRICHTUNG ZUR ERFASSUNG DER HÄMOLYSE ODER ZUR BESTIMMUNG EINES DEN EINFLUSS DER HÄMOLYSE AUF EINE MESSUNG DES HÄMATOKRITS KORRIGIERENDEN KORREKTURFAKTORS**
METHOD AND DEVICE FOR MEASURING THE HEMOLYSIS OR FOR DETERMINING A CORRECTION FACTOR THAT CORRECTS THE INFLUENCE OF THE HEMOLYSIS ON A MEASUREMENT OF THE HEMATOCRIT
PROCÉDÉ ET DISPOSITIF DE DÉTECTION D'HÉMOLYSE OU DE DÉTERMINATION D'UN FACTEUR DE CORRECTION CORRIGEANT L'INFLUENCE DE L'HÉMOLYSE SUR UNE MESURE DE L'HÉMATOCRITE

(30) Priorität: 31.10.2013 DE 102013018284
(43) Veröffentlichungstag der Anmeldung: 07.09.2016
(73) Patentinhaber: Fresenius Medical Care Deutschland GmbH, 61352 Bad Homburg (DE)
(72) Erfinder: WIKTOR, Christoph, 63571 Gelnhausen (DE)
(74) Vertreter: Oppermann, Frank
(86) Internationale Anmeldenummer: PCT/EP2014/073160
(87) Internationale Veröffentlichungsnummer: WO 2015/063113

(56) Entgegenhaltungen:
- EP-A1- 2 199 791
- WO-A1-00/33053
- DE-A1-102009 005 402
- US-B1- 6 294 094
- DENNIS PAUL VALENZENO ET AL: "MEASUREMENT OF CELL LYSIS BY LIGHT SCATTERING", PHOTOCHEMISTRY AND PHOTOBIOLOGY, Bd. 42, Nr. 3, 1. September 1985 (1985-09-01), Seiten 335-339, XP055160336, ISSN: 0031-8655, DOI: 10.1111/j.1751-1097.1985.tb08950.x

## Beschreibung

Die Erfindung betrifft ein Verfahren zur Erfassung der Hämolyse oder zur Bestimmung eines den Einfluss der Hämolyse auf eine Messung des Hämatokrits korrigierenden Korrekturfaktors. Darüber hinaus betrifft die Erfindung eine Vorrichtung zur Erfassung der Hämolyse und zur Bestimmung eines den Einfluss der Hämolyse auf eine Hämatokrit-Messung korrigierenden Korrekturfaktors sowie eine extrakorporale Blutbehandlungsvorrichtung mit einer Vorrichtung zur Erfassung der Hämolyse.

Eine der möglichen Komplikationen bei einer extrakorporalen Blutbehandlung stellt die Hämolyse dar. Unter einer Hämolyse versteht man die Schädigung der roten Blutkörperchen (Erythrozyten). Eine Schädigung der Erythrozyten führt dazu, dass ein Teil des Hämoglobins als freies Hämoglobin an das Blutplasma und ein Teil des Hämoglobins an das im Plasma befindliche Haptoglobin abgegeben wird. Beide Formen des Hämoglobins im Plasma werden als Plasmahämoglobin (PHb) zusammengefasst.

Wenn die Hämoglobinkonzentration im Plasma die des freien Haptoglobins überschreitet, treten klinische Symptome auf, zu denen Schmerzen, Entzündungen und die Ausscheidung von Hämoglobin über den Urin gehören. Weitere Symptome sind erhöhter Blutdruck und Puls, erhöhtes Thromboserisiko, Schluckstörungen und Erbrechen mit Spuren von Blut. Wenn der Hämatokrit infolge einer Hämolyse stark abfällt, kommt es zu einer schlechten Sauerstoffversorgung der Organe und Kurzatmigkeit.

Bei einer Schädigung der Erythrozyten tritt mit dem Hämoglobin das Elektrolyt Kalium aus den Zellen. Zu hohe Kaliumkonzentrationen im Extrazellulärraum (Plasma) stören die Signalweiterleitung in Nerven und Muskeln. Schwere Herzrhythmusstörungen, Muskellähmungen, verminderte Reflexe und vertiefte Atmung sind einige der möglichen Folgen. Da Kalium in der Regel über die Nieren ausgeschieden wird, sind die Folgen einer Hämolyse für Dialysepatienten tendenziell schwerwiegender als für Menschen mit normaler Nierenfunktion. Daher ist die Erfassung der Hämolyse insbesondere bei einer Dialysebehandlung von Bedeutung, bei der die Erythrozyten im extrakorporalen Blutkreislauf zerstört werden können. Mögliche Ursachen für eine Schädigung der Erythrozyten sind Stenosen im extrakorporalen Blutkreislauf aufgrund von Produktionsfehlern, geknickten Schläuchen oder blockierten Kanülen und Kathetern.

Eine Standardmethode zur Erfassung der Hämolyse ist eine optische Messung mit zentrifugierten Plasmaproben, in denen sich die Erythrozyten abgesetzt haben, wobei die Absorption von Licht im Plasma bei verschiedenen Wellenlängen gemessen wird. Der Anteil des freien Hämoglobins im Plasma wird aus den Messwerten mit einer empirisch ermittelten Formel bestimmt.

Die WO 03/100416 A1 beschreibt eine Vorrichtung zum Bestimmen der extrazellulären Hämoglobinkonzentration, die über eine Sende- und eine Empfangseinheit verfügt, die zu beiden Seiten einer Aufnahmeeinheit für eine Blutkonserve angeordnet sind. Mit der Sende- und Empfangseinheit wird die durch das Blut hindurchtretende Transmissionsstrahlung gemessen.

Die WO 2008/000433 A1 beschreibt ein Verfahren und eine Vorrichtung zur Bestimmung der Konzentration von Blutbestandteilen in einer mit Blut gefüllten transparenten Schlauchleitung eines extrakorporalen Blutkreislaufs. Während der Messung ist eine Schlauchleitung eines Schlauchleitungssystems zwischen parallelen, ebenen Anlageflächen eingespannt.

Für Blutbehandlungsvorrichtungen sind austauschbare Einheiten bekannt, die mehrere Komponenten der Blutbehandlungsvorrichtung umfassen. In diesen Kassetten sind auch Kanäle ausgebildet, durch die das Blut des Patienten strömt. Eine derartige Blutkassette ist beispielsweise aus der WO 2010/121819 bekannt.

EP2199791 offenbart eine artverwandte Vorrichtung zum Bestimmen der Hämolyse einer Blutprobe bei der durch die Blutprobe hindurch transmittiertes und / oder von der Blutprobe reflektiertes Messlicht erfasst wird.

Zur Messung des Hämatokrits sind nicht nur Transmissionsmessungen bekannt, sondern auch Verfahren, bei denen die im Blut gestreute Strahlung erfasst wird. Unter Streuung versteht man die Ablenkung der Strahlung durch Wechselwirkung mit einem Streuzentrum, wobei der Streuwinkel als der Winkel definiert ist, um den das gestreute Teilchen abgelenkt wird. Als Vorwärtsstreuung werden Streuprozesse mit einem kleinen Streuwinkel bezeichnet. Rückwärtsstreuung (Reflektion) bezeichnet Streuprozesse mit einem Streuwinkel zwischen 90 Grad und 180 Grad. Bei einer Seitwärtsstreuung ist der Streuwinkel 90 Grad.

Der Erfindung liegt die Aufgabe zu Grunde, ein nicht-invasives Verfahren zur Erfassung der Hämolyse im Vollblut anzugeben, das eine kontinuierliche Messung der Hämolyse erlaubt.

Darüber hinaus ist eine Aufgabe der Erfindung, ein nicht-invasives Verfahren zur Bestimmung eines den Einfluss der Hämolyse auf eine Messung des Hämatokrits korrigierenden Korrekturfaktors anzugeben, der eine genaue Bestimmung des Hämatokrit erlaubt.

Des Weiteren ist eine Aufgabe der Erfindung, eine Vorrichtung zur nicht-invasiven, kontinuierliche Erfassung der Hämolyse oder Bestimmung eines Korrekturfaktors für eine Hämatokrit-Messung zu schaffen. Eine weitere Aufgabe der Erfindung ist, eine extrakorporale Blutbehandlungsvorrichtung, insbesondere Dialysevorrichtung, mit einer Vorrichtung zur Erfassung der Hämolyse bereitzustellen.

Die Lösung dieser Aufgaben erfolgt erfindungsgemäß mit den Merkmalen der unabhängigen Patentansprüche. Die Gegenstände der abhängigen Ansprüche betreffen vorteilhafte Ausführungsformen der Erfindung.

Das erfindungsgemäße Verfahren und die erfindungsgemäße Vorrichtung beruhen auf der Durchführung von zwei unterschiedlichen optischen Messmethoden zur Bestimmung des Hämatokrit, wobei die Hämolyse oder der den Einfluss der Hämolyse auf eine Messung des Hämatokrits korrigierende Korrekturfaktor auf der Grundlage des bei den beiden Messmethoden ermittelten Wertes für den Hämatokrit bestimmt wird. Die beiden Messverfahren können gleichzeitig oder zeitnah durchgeführt werden.

Das Vollblut wird mit einer Strahlung, insbesondere mit Licht bestrahlt, das eine Wellenlänge hat, die vorzugsweise nicht im sichtbaren Bereich (380 nm bis 780 nm) liegt.

Bei der ersten Messmethode wird die in Bezug auf die einfallende Strahlung aus einer ersten Richtung ausfallende Strahlung erfasst, während bei der zweiten Messmethode die in Bezug auf die einfallende Strahlung aus einer zweiten Richtung ausfallende Strahlung erfasst wird, wobei sich die erste Richtung von der zweiten Richtung unterscheidet. Die Intensität der gemessenen Strahlung ist abhängig von dem Hämatokrit. Mit zunehmendem Hämatokrit nimmt die Intensität der Strahlung ab.

Es hat sich gezeigt, dass die mit den unterschiedlichen Messmethoden ermittelten Hämatokritwerte unterschiedlich stark von der ansteigenden Hämoglobinkonzentration im Plasma infolge einer Hämolyse beeinflusst werden. Die Bestimmung der Hämolyse beruht bei dem erfindungsgemäßen Verfahren und der erfindungsgemäßen Vorrichtung darauf, dass eine ansteigende Hämoglobinkonzentration bei der ersten Messmethode zu einer Erhöhung bzw. einer Verringerung des gemessenen Hämatokritwerts und bei der zweiten Messmethode zu einer Verringerung bzw. Erhöhung des Hämatokritwerts führt.

Anstelle der Bestimmung der Hämolyserate erlauben das erfindungs gemäße Verfahren und die erfindungsgemäße Vorrichtung auch die Bestimmung eines Korrekturfaktors, mit dem sich der Einfluss der Hämolyse auf eine Messung des Hämatokrits mit den bekannten Hämatokrit-Messverfahren korrigieren lässt, bei denen der Einfluss der Hämolyse keine Berücksichtigung findet. Dabei wird in der Regel davon ausgegangen, dass mit zunehmender Konzentration des freien Hämoglobins im Plasma bzw. zunehmender Hämolyserate der Fehler der Messung mit den bekannten Hämatokrit-Messverfahren zunimmt. Unter einem Korrekturfaktor wird auch ein Korrekturwert verstanden, d.h. ein absoluter Betrag, um den der gemessene Wert erhöht oder verringert wird.

Die erfindungsgemäße Vorrichtung zur Bestimmung des Korrekturfaktors kann eine Baugruppe der eigentlichen Vorrichtung zur Messung des Hämatokrits bilden.

Die Hämolyse oder der Korrekturfaktor wird auf der Grundlage der Differenz des nach der ersten und zweiten Messmethode ermittelten Wertes für den Hämatokrit bestimmt. Es hat sich gezeigt, dass mit zunehmender Differenz zwischen beiden gemessenen Hämatokritwerten die Hämolyse zunimmt. Folglich ist die Differenz der Messwerte ein Maß für die Hämolyse.

Eine weitere besonders bevorzugte Ausführungsform sieht vor, die Hämolyserate auf der Grundlage eines vorgegebenen Zusammenhangs zwischen der Differenz des nach der ersten und zweiten Messmethode ermittelten Wertes für den Hämatokrit und der Hämolyserate zu bestimmen. Unterschiedlichen Differenzen der Hämatokritwerte können jeweils eine bestimmte Hämolyserate zugeordnet werden. Die empirisch ermittelten Wertepaare können in einem Speicher gespeichert werden. Die Hämolyserate kann aber auch auf der Grundlage einer vorgegebenen Funktion berechnet werden, die den Zusammenhang zwischen den Differenzwerten und der Hämolyserate beschreibt.

Die Messmethoden unterscheiden sich in der Erfassung der Transmissionsstrahlung oder Streustrahlung voneinander. Als Streustrahlung kann die Vorwärtsstreuung, Rückwärtsstreuung (Reflektion) oder Seitwärtsstreuung erfasst werden.

In Versuchen hat sich gezeigt, dass bei einer Transmissionsmessung mit zunehmender Konzentration von freiem Hämoglobin im Plasma kleinere Hämatokritwerte gemessen werden als bei einer Streuungsmessung. Bei der Streuungsmessung ergeben sich unterschiedliche Abhängigkeiten zwischen dem gemessenen Hämatokritwert und der Konzentration von freiem Hämoglobin im Plasma. Bei der Reflektionsmessung zeigt sich die stärkste Abhängigkeit. Während bei der Transmissionsmessung die gemessenen Hämatokritwerte mit ansteigender Konzentration des freien Hämoglobins im Plasma abnehmen, nehmen die mit der Reflektionsmessung gemessenen Hämatokritwerte mit steigender Plasma-Hämoglobinkonzentration zu. Es hat sich für sämtliche Messmethoden ein linearer Zusammenhang zwischen den gemessenen Hämatokritwerten und der Plasma-Hämoglobinkonzentration gezeigt.

Eine besonders bevorzugte Ausführungsform der Erfindung sieht als erste Messmethode eine Reflektionsmessung und als zweite Messmethode eine Transmissionsmessung vor. Die Erfassung der Hämolyse erfolgt dann auf der Grundlage einer Differenzbildung zwischen den Hämatokritwerten bei der Reflektions- und der Transmissionsmessung.

Die erfindungsgemäße Vorrichtung verfügt über eine Sende- und Empfangseinheit zum Bestrahlen des Vollblutes und zum Empfangen der aus dem Vollblut austretenden oder reflektierten Strahlung nach einer der beiden Messmethoden, insbesondere der Transmissions- und Reflektionsstrahlung. Darüber hinaus verfügt die erfindungsgemäße Vorrichtung über eine Rechen- und Auswerteinheit, die derart konfiguriert ist, dass die Hämolyse oder der Korrekturfaktor auf der Grundlage des nach der ersten und zweiten Messmethode ermittelten Wertes für den Hämatokrit bestimmt wird. Die Rechen- und Auswerteinheit ist vorzugsweise eine Datenverarbeitungseinheit (Mikroprozessor), auf der ein Datenverarbeitungsprogramm läuft.

Bei einer bevorzugten Ausführungsform weist die erfindungsgemäße Vorrichtung eine Einheit mit einem Aufnahmeraum zur klemmenden Aufnahme einer Schlauchleitung eines extrakorporalen Blutkreislaufsauf. Eine alternative Ausführungsform sieht eine Einheit zur Befestigung einer Kassette vor, die einen Kanal aufweist, in dem das Blut strömt.

Die Sende- und Empfangseinheit weist einen oder mehrere Sender und einen oder mehrere Empfänger auf, die auf einer der Seiten der Aufnahmeeinheit angeordnet sind. Bei einer ersten alternativen Ausführungsform verfügt die Sende- und Empfangseinheit über nur einen Sender und zwei Empfänger, während eine zweite alternative Ausführungsform über zwei Sender und nur einen Empfänger verfügt. Während bei der ersten Ausführungsform die Strahlung des einzigen Senders von den beiden Empfängern gleichzeitig aus den unterschiedlichen Richtungen empfangen werden kann, müssen bei der zweiten Ausführungsform die beiden Sender abwechselnd ein- bzw. ausgeschaltet werden, um mit dem einzigen Empfänger die Strahlung aus den unterschiedlichen Richtungen empfangen zu können. Es ist aber auch möglich, dass die Sende- und Empfangseinheit für jede der beiden Messmethoden jeweils einen Sender und einen Empfänger aufweist. Hierbei können die Sender und Empfänger jeweils eine oder mehrere LED's bzw. Fotodioden umfassen.

Die erfindungsgemäße Vorrichtung kann eine separate Baugruppe bilden oder Bestandteil einer extrakorporalen Blutbehandlungsvorrichtung, insbesondere einer Dialysevorrichtung sein. Wenn die erfindungsgemäße Vorrichtung Bestandteil der Blutbehandlungsvorrichtung ist, kann die Rechen- und Auswerteinheit der erfindungsgemäßen Vorrichtung auch Bestandteil der zentralen Datenverarbeitungseinheit der Blutbehandlungsvorrichtung sein.

Mit der erfindungsgemäßen Vorrichtung wird vorzugsweise die Hämolyserate in der venösen Blutleitung gemessen, die von der Austauscheinheit der extrakorporalen Blutbehandlungsvorrichtung zum Patienten führt, da die Hämolyserate infolge einer mechanischen Schädigung der Erythrozyten im extrakorporalen Blutkreislauf beim Eintritt des Blutes in den Patienten nach Durchlaufen des extrakorporalen Kreislaufs am höchsten ist. Es ist aber auch eine Messung in der arteriellen Blutleitung möglich. Die Kombination beider Messungen erlaubt die Überwachung des gesamten extrakorporalen Blutkreislaufs.

Im Folgenden wird ein Ausführungsbeispiel der Erfindung unter Bezugnahme auf die Zeichnungen im Einzelnen beschrieben.

Es zeigen:
- Fig. 1: eine Vorrichtung zur extrakorporalen Blutbehandlung zusammen mit einer Vorrichtung zur Erfassung der Hämolyse in stark vereinfachter schematischer Darstellung,
- Fig. 2A: eine Teilansicht der Messeinheit der Vorrichtung zur Erfassung der Hämolyse mit einem Sender und Empfänger für die Erfassung der Streustrahlung in vereinfachter Darstellung,
- Fig. 2B: eine Teilansicht der Messeinheit mit einem Sender und Empfänger für die Erfassung der Transmissionsstrahlung in vereinfachter Darstellung,
- Fig. 2C: eine Teilansicht einer alternativen Ausführungsform der Messeinheit mit einem Sender und Empfänger für die Erfassung der Streustrahlung in vereinfachter Darstellung,
- Fig. 3: eine Prinzipdarstellung der Sende- und Empfangseinheit zur Messung der Strahlung nach den unterschiedlichen Messmethoden,
- Fig. 4: die Intensität des Messsignals als Funktion des Hämatokrits,
- Fig. 5 A bis Fig. 5 D: die Abhängigkeit des Messsignals vom Hämatokrit bei unterschiedlichen Messmethoden,
- Fig. 6: die Abhängigkeit des mit den unterschiedlichen Messmethoden bestimmten Hämatokritwertes von der Plasmahämoglobinkonzentration,
- Fig. 7: die Abhängigkeit der Differenz der mit einer Reflektions- und Transmissionsmessung ermittelten Hämatokritwerte von der Hämolyserate,
- Fig. 8: ein erstes Ausführungsbeispiel einer Messanordnung für eine Reflektions- und Transmissionsmessung und
- Fig. 9: ein zweites Ausführungsbeispiel einer Messanordnung für eine Reflektions- und Transmissionsmessung.

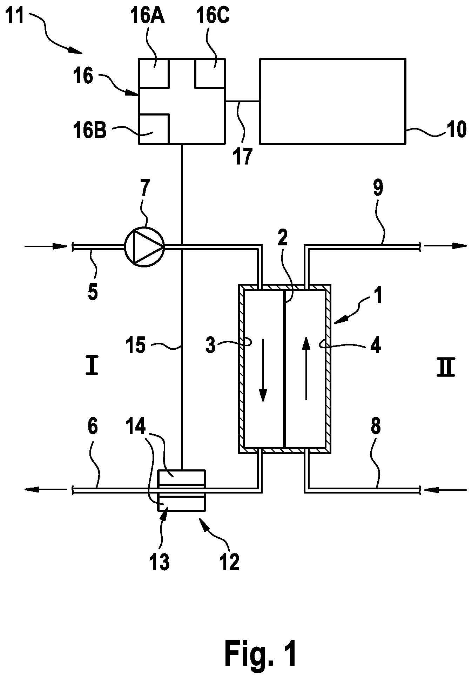

Fig. 1 zeigt nur die für die Erfindung wesentlichen Komponenten einer Vorrichtung zur extrakorporalen Blutbehandlung, insbesondere einer Dialysevorrichtung, in stark vereinfachter schematischer Darstellung. Die extrakorporale Blutbehandlungsvorrichtung verfügt über eine Austauscheinheit, beispielsweise einen Dialysator oder Filter 1, der durch eine semipermeable Membran 2 in eine Blutkammer 3 und eine Dialysierflüssigkeitskammer 4 unterteilt ist. Von dem Patienten führt eine arterielle Blutzuführleitung 5 zu der Blutkammer des Dialysators 1, während von der Blutkammer eine venöse Blutrückführleitung 6 abgeht, die zu dem Patienten führt. Eine in der arteriellen Blutleitung 5 angeordnete Blutpumpe 7 fördert das Blut im extrakorporalen Blutkreislauf I.

Das Dialysierflüssigkeitssystem II der Dialysevorrichtung ist nur andeutungsweise dargestellt. Es umfasst eine zu der Dialysierflüssigkeitskammer 4 führende Dialysierflüssigkeitszuführleitung 8 und eine von der Dialysierflüssigkeitskammer 4 des Dialysators 1 abgehende Dialysierflüssigkeitsabführleitung 9. Bei der arteriellen und venösen Blutleitung 5, 6 handelt es sich um Schlauchleitungen, die für elektromagnetische Strahlung, insbesondere für Licht, zumindest teilweise durchlässig sind.

Darüber hinaus verfügt die Blutbehandlungsvorrichtung über eine zentrale Steuereinheit 10, mit der die einzelnen Komponenten, beispielsweise die Blutpumpe 7, gesteuert werden. Die erfindungsgemäße Vorrichtung 11 zum Bestimmen der Hämolyse ist bei dem vorliegenden Ausführungsbeispiel Bestandteil der Blutbehandlungsvorrichtung, so dass sie von Komponenten Gebrauch machen kann, die in der Blutbehandlungsvorrichtung ohnehin vorhanden sind.

Die Vorrichtung 11 zum Erfassen der Hämolyse oder des Korrekturfaktors verfügt über eine Messeinheit 12, die eine Einheit 13 umfasst, in die sich eine Schlauchleitung des extrakorporalen Blutkreislaufs, insbesondere die venöse Blutleitung 6, passend einlegen lässt. Die Messeinheit 12 umfasst weiterhin eine Sende- und Empfangseinheit 14 zum Ein- und Auskoppeln der Strahlung.

Die Messeinheit 12 ist über eine Datenleitung 15 mit einer Rechen- und Auswerteinheit 16 verbunden. Die Rechen- und Auswerteinheit 16 kann die Daten über eine Leitung 17 mit der zentralen Steuereinheit 10 der Blutbehandlungsvorrichtung austauschen.

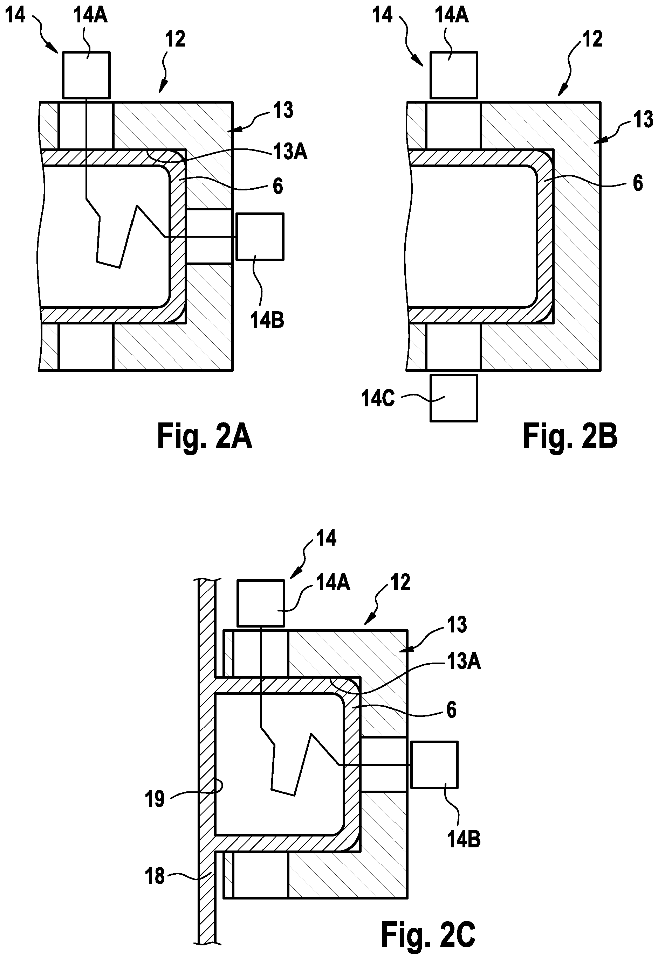

Fig. 2A zeigt eine Teilansicht der Messeinheit 12 in vereinfachter geschnittener Darstellung. Die Messeinheit 12 weist eine Einheit 13 mit einem Aufnahmeraum 13A auf, in dem die Schlauchleitung 6 eingespannt ist. Der Aufnahmeraum 13A weist vier rechtwinklig zueinander stehende, ebene Anlageflächen auf, an denen die Schlauchleitung anliegt. In Fig. 2A sind nur ein Sender 14A und ein Empfänger 14B der Sende- und Empfangseinheit 14 dargestellt. Die von dem Sender 14A emittierte Strahlung tritt durch die Schlauchleitung 6 in das in der Schlauchleitung strömende Blut ein, wobei die aus dem Blut austretende Strahlung durch die Schlauchleitung auf den Empfänger 14B trifft. Da die Achsen von Sender- und Empfänger 14A, 14B in einem rechten Winkel zueinander angeordnet sind, empfängt der Empfänger die Streustrahlung. Für die Erfassung der Transmissionsstrahlung liegen sich Sender und Empfänger 14A, 14C auf einer gemeinsamen Achse einander gegenüber, was in Fig. 2B dargestellt ist.

Fig. 2C zeigt eine alternative Ausführungsform der Messeinheit 12, die für eine Blutkassette 18 bestimmt ist, bei der das Blut nicht in einer Schlauchleitung, sondern in einem Blutkanal 19 strömt, der in der Kassette ausgebildet ist. Der Teil der Blutkassette 18 mit dem Kanal 19 besteht aus transparentem Material. Die Messeinheit 12 weist eine beispielsweise an einer Seite geöffnete Einheit 13 auf, an der sich die Kassette 18 befestigen lässt oder die sich an der Kassette befestigen lässt. Messeinheit 12 und Kassette 18 bilden somit separate Einheiten, wobei die Messeinheit 12 Bestandteil der Blutbehandlungsvorrichtung und die Kassette 18 austauschbar ist.

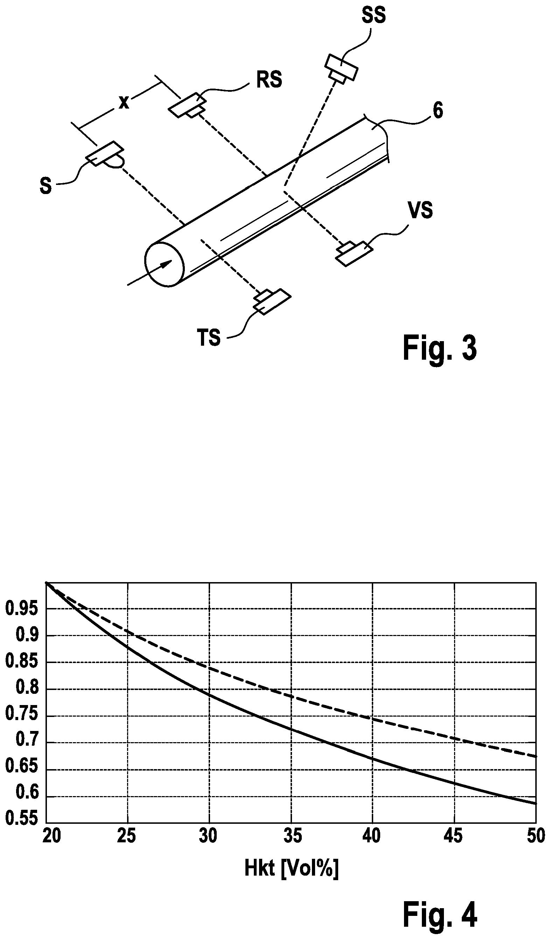

Fig. 3 zeigt eine Prinzipdarstellung einer Messanordnung mit einer Sende- und Empfangseinheit 14, die mehrere Sender und Empfänger umfasst, um nach unterschiedlichen Messmethoden den Hämatokrit bestimmen zu können. Da die einzelnen Messmethoden zum Stand der Technik gehören, wird nur das Prinzip der Messanordnung beschrieben.

Das Blut strömt in der transparenten Schlauchleitung 6, die in der in Fig. 3 nicht dargestellten Einheit 13 der Vorrichtung zur Bestimmung der Hämolyse eingespannt ist. Die Messanordnung für die Transmissionsmessung weist einen Sender S und einen Empfänger auf, die zu beiden Seiten der Schlauchleitung sich auf einer gemeinsamen Achse einander gegenüber liegen. Der Empfänger für die Erfassung der transmittierten Strahlung ist mit TS bezeichnet. Die Achse von Sender S und Empfänger TS verläuft im rechten Winkel zur Längsachse der Schlauchleitung 6. Das Licht des Senders S, das sich in Richtung der Achse ausbreitet und auf das in die Schlauchleitung strömende Blut trifft, wird von dem Empfänger TS empfangen. Der Empfänger TS liefert ein mit der Intensität des Lichts proportionales Messsignal, das in der Rechen- und Auswerteinheit 16 ausgewertet wird. Der Zusammenhang zwischen der Intensität des einfallenden und ausfallenden Lichts beschreibt das bekannte Lambert-Beersche Gesetz, nach dem der Hämatokrit in der Rechen- und Auswerteinheit 16 berechnet wird.

Die Messanordnung weist für die Erfassung der Streustrahlung (Streulichtmessung) drei weitere Empfänger auf. Der Empfänger für die Erfassung der Rückwärtsstreuung (Reflektion) ist mit RS, der Empfänger zur Erfassung der Vorwärtsstreuung mit VS und der Empfänger zur Erfassung der Seitwärtsstreuung mit SS bezeichnet. Die Empfänger RS und VS sind im Abstand x zu dem Sender S und Empfänger TS für die Transmissionsmessung angeordnet. Der Sender S und der Empfänger TS für die Transmissionsmessung und die Empfänger RS und VS für die Erfassung der Rückwärtsstreuung und Vorwärtsstreuung sind in einer Ebene angeordnet, durch die sich die Längsachse der Schlauchleitung 6 erstreckt. Der Empfänger RS zur Erfassung der Rückwärtsstreuung, der Empfänger VS zur Erfassung der Vorwärtsstreuung und der Empfänger SS zur Erfassung der Seitwärtsstreuung liegen in einer dazu senkrecht stehenden Ebene. Zur Erfassung der Seitwärtsstreuung kann der Abstand x auch gleich Null sein.

Die Wellenlänge der von dem Sender S ausgesandten Strahlung, insbesondere des ausfallenden Lichts, liegt vorzugsweise nicht im sichtbaren Bereich von 380 nm bis 780 nm. Der Sender ist vorzugsweise eine schmalbandige LED mit einer Spitzenwellenlänge, die bei 805 nm liegt. Die Empfänger sind Fotodioden.

Fig. 4 zeigt die Änderung der Intensität des Signals des Empfängers SS (Relatives Messsignal (x = 0)) für die Seitwärtsstreuung als Funktion des Hämatokrit Hkt in Vol%, wobei als Sender eine LED mit einem kleinen Abstrahlwinkel von 30 Grad und einem großen Abstrahlwinkel von 110 Grad verwendet wird. Das Messsignal der LED (SMD LED) mit einem Abstrahlwinkel von 30 Grad ist in durchgezogener Linie und das Messsignal der LED (Weitwinkel LED (5 mm)) mit einem Abstrahlwinkel von 110 Grad in gestrichelter Linie dargestellt. Es zeigt sich, dass die Änderung des Messsignals mit zunehmendem Hämatokrit bei der LED mit einem kleinen Abstrahlwinkel größer als mit der LED ist, die einen großen Abstrahlwinkel hat. Zur Erhöhung der Empfindlichkeit sollte der Abstrahlwinkel der LED also klein sein.

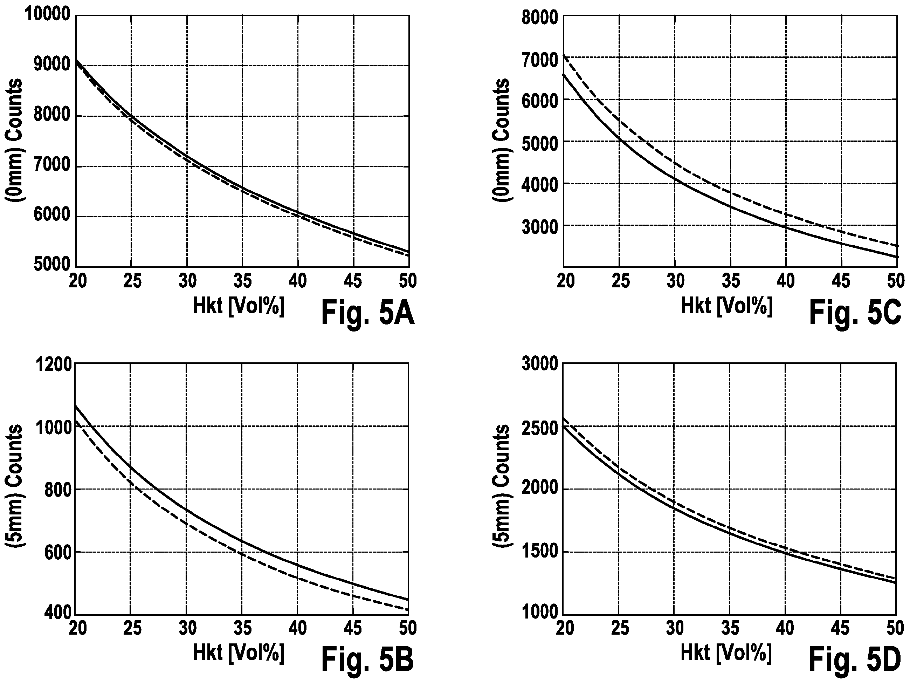

Die Figuren 5A bis 5D zeigen die Abhängigkeit der Intensität der mit den unterschiedlichen Messmethoden gemessenen Intensität der Strahlung [Counts] von dem Hämatokrit Hkt [Vol%], wobei die Messerwerte für Menschenblut in durchgezogener und die für Rinderblut in gestrichelter Linie dargestellt sind. Für sämtliche Messmethoden zeigt sich, dass die Intensität der Strahlung mit zunehmendem Hämatokrit abnimmt.

Fig. 5A zeigt die Abhängigkeit der Strahlungsintensität von dem Hämatokrit Hkt für die Messmethode, bei der die Seitwärtsstreuung (Seitwärtstreuung in Counts) erfasst wird, wobei der Abstand x=0 ist. Fig. 5B zeigt die Abhängigkeit der Seitwärtsstreuung (Seitwärtstreuung in Counts) von dem Hämatokrit bei einem Abstand x=5 mm. Fig. 5C zeigt die Abhängigkeit der Intensität der mit der Transmissionsmessung (x=0 mm) und Fig. 5D die Abhängigkeit der Intensität der mit der Reflektionsmessung (x=5 mm) gemessenen Streustrahlung von dem Hämatokrit Hkt. Es zeigt sich eine unterschiedliche Abhängigkeit der Strahlungsintensität von dem Hämatokrit für die unterschiedlichen Messmethoden (Seitwärtsstreuung, Transmission, Reflektion).

Der mit den unterschiedlichen Messmethoden ermittelte Wert für den Hämatokrit ist von der Konzentration des freien Hämoglobins im Plasma infolge der Hämolyse abhängig. Für die unterschiedlichen Messmethoden zeigen sich unterschiedliche Abhängigkeiten. Es zeigt sich aber immer ein linearer Zusammenhang zwischen dem Hämatokrit und der Konzentration des freien Hämoglobins.

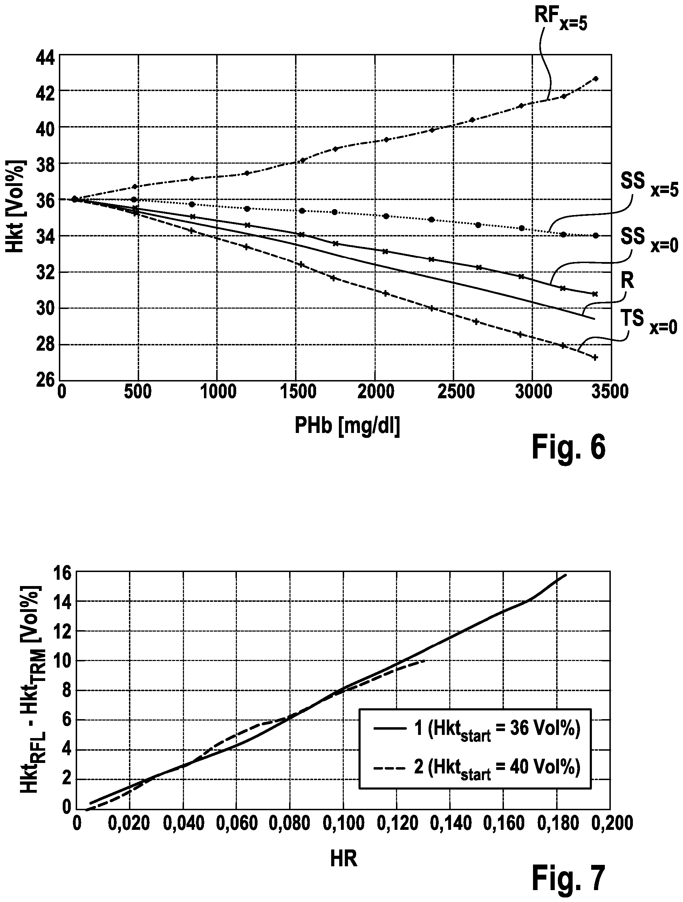

Die Abhängigkeit des Hämatokrits von der Konzentration des freien Hämoglobins im Plasma ist für die unterschiedlichen Messmethoden in Fig. 6 dargestellt. Die Grafen in Fig. 6 sind wie folgt bezeichnet:
- RF:: Referenzwert
- SSₓ₌₀: Seitwärtsstreuung (x=0 mm),
- TSₓ₌₀: Transmission (x=0mm),
- SSₓ₌₅: Seitwärtsstreuung (x=5mm),
- Rₓ₌₅: Reflektion (x=5mm).

Auf der y-Achse ist der Hämatokrit HKt in Vol % und auf der x-Achse die Konzentration K des freien Hämoglobins im Plasma in mg/dl aufgetragen.

Bei der Reflektionsmessung nehmen die gemessenen Hämatokritwerte mit zunehmender Konzentration des freien Hämoglobins im Plasma zu, während bei der Messung der Seitwärtsstreuung und der Transmission die gemessenen Hämatokritwerte mit zunehmender Plasma-Hämoglobinkonzentration abnehmen. Die stärkste Abnahme der gemessenen Hämatokritwerte zeigt sich bei der Transmissionsmessung. Es zeigt sich, dass die Streuungsmessung den Hämatokrit mit zunehmender Hämoglobinkonzentration im Plasma überschätzt, während die Transmissionsmessung den Hämatokrit unterschätzt. Die Reflektionsmessung reagiert am empfindlichsten auf die ansteigende Hämolyse.

Bei dem vorliegenden Ausführungsbeispiel wird der Hämatokrit mit zwei unterschiedlichen Messmethoden bestimmt. Die erste Messmethode erfolgt mit einer Reflektionsmessung und die zweite Messmethode mit einer Transmissionsmessung. Bei diesen beiden Messmethoden zeigt sich die größte Abhängigkeit des gemessenen Hämatokritwertes von der Konzentration des freien Hämoglobins im Plasma.

Die Rechen- und Auswerteinheit 16 berechnet die Differenz zwischen dem mit der Reflektionsmessung gemessenen Hämatokritwert Hkt_{RFL} und dem mit der Transmissionsmessung gemessenen Hämatokritwert Hkt_{TRM}, wobei auf der Grundlage der Differenz beider Messwerte auf das Vorliegen einer Hämolyse und die Höhe der Hämolyserate geschlossen wird. Mit zunehmender Differenz wird auf eine Erhöhung der Hämolyserate geschlossen.

Die Hämolyserate HR ist eine vom Hämatokrit unabhängige dimensionslose Größe zur Bestimmung der Hämolyse. Sie berechnet sich aus HR=(100%-Hkt)^{∗}PHb/GHb, wobei PHb die Hämoglobinkonzentration im Plasma und GHb die Gesamthämoglobinkonzentration im Vollblut ist.

Fig. 7 zeigt, dass zwischen der Differenz Hkt_{RFL} - Hkt_{TRM} der gemessenen Messwerte Hkt_{RFL}, Hkt_{TRM} für den Hämatokrit und der Hämolyserate ein weitgehend linearer Zusammenhang besteht. Die Messwerte wurden in zwei Versuchen ermittelt. Der in durchgezogener Linie dargestellte Graf zeigt die Abhängigkeit der Differenz der gemessenen Hämatokritwerte von der Hämolyserate für einen Startwert des Hämatokrit von 36 Vol % und der in gestrichelter Linie dargestellte Graf für einen Startwert des Hämatokrit 40 Vol %. Beide Grafen lassen sich durch eine Gerade approximieren.

Die einzelnen Differenzmesswerte Hkt_{RFL} - Hkt_{TRM} und die zugehörigen Hämolyseraten HR sind in einem Speicher der Rechen- und Auswerteinheit 16 abgelegt. Die Rechen- und Auswerteinheit 16 liest aus dem Speicher 16A für die ermittelte Differenz zwischen den Hämatokritwerten die Hämolyserate HR aus. Beispielsweise ergibt sich bei einer Differenz der Messwerte von 8 Vol % eine Hämolyserate HR von 100. In der Rechen- und Auswerteinheit 16 kann aber auch eine die Abhängigkeit der Differenz der Hämatokritwerte von der Hämolyserate beschreibende Gleichung abgelegt sein, mit der die Hämolyserate in Abhängigkeit von der Differenz der gemessenen Hämatokritwerte berechnet wird.

Die Hämolyserate wird auf einer Anzeigeeinheit 16B der Vorrichtung 11 zur Erfassung der Hämolyse dargestellt. Die Vorrichtung kann über eine Alarmeinheit 16C verfügen, die bei Überschreiten einer vorgegebenen Hämolyserate Alarm gibt. Bei Überschreiten einer vorgegebenen Hämolyserate kann auch ein Steuersignal erzeugt werden, das die zentrale Steuereinheit 10 der Blutbehandlungsvorrichtung über die Leitung 17 empfängt, so dass ein Eingriff in die Maschinensteuerung der Blutbehandlungsvorrichtung vorgenommen werden kann.

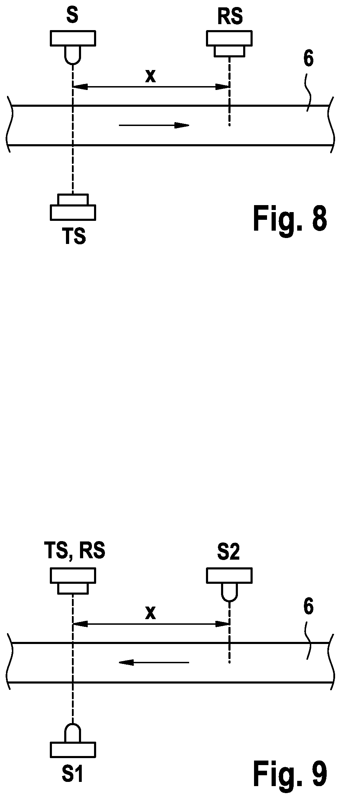

Das bevorzugte Ausführungsbeispiel der Erfindung macht nur von einer Reflektions- und Transmissionsmessung Gebrauch. Fig. 8 zeigt eine erste alternative Ausführungsform der Messanordnung für die Reflektions- und Transmissionsmessung. Dieses Ausführungsbeispiel entspricht der in Fig. 3 gezeigten Ausführungsform, wobei die Empfänger VS und SS für die Vorwärts- und Seitwärtsstreuung entfallen. Die einander entsprechenden Teile sind daher mit den gleichen Bezugszeichen versehen. Die Messanordnung von Fig. 8 erlaubt beiden Empfängern TS und RS die gleichzeitige Messung der reflektierten bzw. transmittierten Strahlung. Fig. 9 zeigt eine alternative Ausführungsform, die über eine Messstrecke für die Transmissionsmessung mit einem Sender S1 und einem Empfänger TS, RS verfügt, der auch für die Reflektionsmessung verwendet wird. Für die Reflektionsmessung ist ein zweiter Sender S2 vorgesehen, der im Abstand x der Messstrecke für die Transmissionsmessung angeordnet ist. Diese Messanordnung erlaubt nicht eine gleichzeitige, sondern nur eine abwechselnde Messung mit den beiden Messmethoden. Für die Transmissionsmessung schaltet die Rechen- und Auswerteinheit 16 den Sender S1 für die Transmissionsmessung ein und den Sender S2 für die Reflektionsmessung aus, während für die Reflektionsmessung der Sender S1 für die Transmissionsmessung ausgeschaltet und der Sender S2 für die Reflektionsmessung eingeschaltet wird. Beide Messungen sollten unmittelbar aufeinander erfolgen.

Die erfindungsgemäße Vorrichtung erlaubt eine nicht-invasive, kontinuierliche Erfassung der Hämolyse im Vollblut, unabhängig vom Hämatokrit und von der Sauerstoffsättigung. Sie zeichnet sich durch einen einfachen apparativen Aufbau und eine einfache Auswertung der Messergebnisse aus. Die erfindungsgemäße Vorrichtung kann in sämtlichen Blutbehandlungsvorrichtungen verwendet werden, die über einen extrakorporalen Blutkreislauf verfügen. Mit der erfindungsgemäßen Vorrichtung kann zur Qualitätssicherung auch die Hämolyse in Vollblut erfasst werden, das sich in Blutkonserven befindet. Hierfür kann die Aufnahmeeinheit zur Aufnahme einer Blutkonserve oder eines Schlauchs an der Blutkonserve ausgebildet sein.

Ein anderer Aspekt der Erfindung liegt in der Bestimmung eines Korrekturfaktors für eine Messung des Hämatokrits mit einem konventionellen optischen Hämatokrit-Messverfahren, bei dem der Einfluss der Hämolyse keine Berücksichtigung findet.

Fig. 6 zeigt die Abhängigkeit des mit den unterschiedlichen Messmethoden (RF ₓ₌₅, Reflektion (x=5mm), SSₓ₌₅ Seitwärtsstreuung (x=5mm), SSₓ₌₀ Seitwärtsstreuung (x=0 mm), TSₓ₌₀ Transmission (x=0mm)) bestimmten Hämatokrits Hkt von der Hämoglobinkonzentration im Plasma PHb für einen Startwert des Hämatokrits von 36 %, bei dem die Hämoglobinkonzentration im Plasma PHb klein ist. Der Fehler der Hämatokrit-Messung nimmt mit zunehmender Konzentration des freien Hämoglobins im Plasma zu. Die Kurve R zeigt den aus einer Referenzmessung bestimmten, tatsächlichen Hämatokrit für diese Messreihe.

Es hat sich gezeigt, dass die Steigung der Kurven gleich bleibt, wenn sich der Startwert für den Hämatokrit, der in Fig. 6 bei 36 % liegt, in einem relativ weiten Bereich verändert. Die Kurve für einen anderen Startwert als 36 % kann einfach dadurch ermittelt werden, dass die Kurve für den Startwert von 36 % parallel zu dem neuen Startwert verschoben wird. Eine Veränderung des Startwerts für den Hämatokrit führt also nur zu einer Parallelverschiebung der Kurven. Folglich legt die Differenz der mit der Reflektions- und Transmissionsmessung gewonnenen Messwerte Hkt_{RFL} - Hkt_{TRM} das freie Hämoglobin im Plasma (X-Koordinate) weitgehend unabhängig vom Startwert fest.

Fig. 6 zeigt für die Referenzkurve R eine Abhängigkeit von der Hämoglobinkonzentration im Plasma PHb. Die Referenzkurve R ist aber in der Realität bei volumetrischen Messungen am Patientenblut unabhängig vom freien Hämoglobin, das keinen Volumenanteil beiträgt. Die Abhängigkeit von R(PHb), die sich in Figur 6 zeigt, ist so zu erklären, dass die gezeigten Kurven aus einem Laborversuch resultieren, bei dem hämolisiertes Vollblut der Probe hinzugefügt wurde. Dieses besteht aus freiem Hämoglobin und Blutplasma, so dass sich der Anteil des Hämatokrit an der Probe durch den gestiegenen Anteil des Plasmas insgesamt verändert. Der Abstand der Kurven untereinander bleibt aber auch bei Messungen am Patienten unverändert. Der tatsächliche Kurvenverlauf, der sich bei Messungen am Patienten ergeben würde, ist also der in Fig. 6 gezeigte Kurvenverlauf, bei dem alle Kurven um ihren gemeinsamen linken Anknüpfungspunkt so gegen den Uhrzeigersinn gedreht werden, dass die Referenzkurve R eine horizontale Linie bildet, so dass Hkt unabhängig von PHb ist. Für die Bestimmung des Korrekturwertes ist dies aber unerheblich.

Die empirisch ermittelte Kurvenschar von Figur 6 ist in der Rechen- und Auswerteinheit 16 vorzugsweise nur für einen Startwert des Hämatokrit Hkt, beispielsweise Hkt= 36 %, oder für eine Vielzahl von Startwerten gespeichert. Wenn die Kurvenschar nur für einen Startwert des Hämatokrit Hkt gespeichert ist, kann aus der Differenz der gemessenen Werte Hkt des Hämatokrit aber dennoch eindeutig die Hämoglobinkonzentration im Plasma PHb abgeleitet werden, da die Plasmahämoglobinkonzentration im Wesentlichen nur von der Differenz der Messwerte abhängig ist.

Es sei angenommen, dass mit der Reflektionsmessung (RF ₓ₌₅) Hkt_{RFL} ein Hämatokrit Hkt von 40 % und mit der Transmissionsmesssung (TSₓ₌₀) ein Hämatokrit Hkt von 29,5 % gemessen wird. Für die beiden Messungen ergibt sich eine Hämoglobinkonzentration im Plasma PHb von 2500 mg/dl (Fig. 6). Der tatsächliche Hämatokrit (31 %) kann dann mit der Referenzmessung ermittelt werden, bei der für eine Hämoglobinkonzentration im Plasma PHb von 2500 mg/dl der Hämatokrit Hkt 31 % ist. Die gemessenen Werte für den Hämatokrit von 40 % bzw. 29,5 % müssen also um 9 % bzw. - 1,5 % korrigiert werden, was dem Abstand der Kurve für die Reflektionsmessung (RF ₓ₌₅) von der Kurve für die Referenzmessung (R) bzw. dem Abstand der Kurve für die Transmissionsmessung (TSₓ₌₀) von der Kurve für die Referenzmessung (R) entspricht. Es ergeben sich also für beide Messungen Korrekturfaktoren von 9 % bzw. - 1,5 %. Diese Korrekturfaktoren sind von der Hämoglobinkonzentration im Plasma PHb abhängig und werden kleiner, wenn die Hämoglobinkonzentration im Plasma PHb abnimmt.

## Patentansprüche

1. Verfahren zur Messung der Hämolyse im Vollblut oder Bestimmung eines den Einfluss der Hämolyse auf eine Messung des Hämatokrits korrigierenden Korrekturfaktors mit folgenden Verfahrensschritten:
Bestrahlen des Vollblutes mit einer auf das Vollblut gerichteten optischen Strahlung,
Erfassen einer in Bezug auf die einfallende Strahlung aus einer ersten Richtung ausfallenden Strahlung zur Durchführung einer ersten Messung nach einer ersten Messmethode und
Erfassen einer in Bezug auf die einfallende Strahlung aus einer zweiten Richtung ausfallenden Strahlung zur Durchführung einer zweiten Messung nach einer zweiten Messmethode,
Bestimmen eines ersten Wertes für den Hämatokrit aus der Intensität der aus der ersten Richtung ausfallenden Strahlung nach der ersten Messmethode,
Bestimmen eines zweiten Wertes für den Hämatokrit aus der Intensität der aus der zweiten Richtung ausfallenden Strahlung nach der zweiten Messmethode,
**dadurch gekennzeichnet, dass**
die Hämolyse oder der den Einfluss der Hämolyse auf die Messung des Hämatokrits korrigierende Korrekturfaktor auf der Grundlage des nach der ersten und zweiten Messmethode ermittelten Wertes für den Hämatokrit bestimmt wird, wobei die Hämolyse oder der den Einfluss der Hämolyse auf die Messung des Hämatokrits korrigierende Korrekturfaktor auf der Grundlage der Differenz des nach der ersten und zweiten Messmethode ermittelten Wertes für den Hämatokrit bestimmt wird, wobei die aus der ersten Richtung ausfallende Strahlung die in dem Vollblut gestreute Streustrahlung ist und die aus der zweiten Richtung ausfallende Strahlung die durch das Vollblut hindurchtretende Transmissionsstrahlung ist, die in der Richtung der auf das Vollblut gerichteten Strahlung gerichtet ist.

2. Verfahren nach Anspruch 1, **dadurch gekennzeichnet, dass** die Hämolyserate auf der Grundlage eines vorgegebenen linearen Zusammenhangs zwischen der Differenz des nach der ersten und zweiten Messmethode ermittelten Wertes für den Hämatokrit und der Hämolyserate bestimmt wird.

3. Verfahren nach Anspruch 1 oder 2, **dadurch gekennzeichnet, dass** die aus der ersten Richtung ausfallende Strahlung die rückwärtsgestreute Streustrahlung ist, die der Richtung der auf das Vollblut gerichteten Strahlung entgegen gerichtet ist.

4. Vorrichtung zur Messung der Hämolyse im Vollblut oder Bestimmung eines den Einfluss der Hämolyse auf eine Messung des Hämatokrits korrigierenden Korrekturfaktors mit
einer Sende- und Empfangseinheit (14) zum Bestrahlen des Vollblutes mit einer auf das Vollblut gerichteten optischen Strahlung und zum Erfassen einer in Bezug auf die einfallende Strahlung aus einer ersten Richtung ausfallenden Strahlung zur Durchführung einer ersten Messung nach einer ersten Messmethode und zum Erfassen einer in Bezug auf die einfallende Strahlung aus einer zweiten Richtung ausfallenden Strahlung zur Durchführung einer zweiten Messung nach einer zweiten Messmethode,
einer Rechen- und Auswerteinheit (16) die derart konfiguriert ist, dass ein erster Wert für den Hämatokrit aus der Intensität der aus der ersten Richtung ausfallenden Strahlung nach der ersten Messmethode und ein zweiter Wert für den Hämatokrit aus der Intensität der aus der zweiten Richtung ausfallenden Strahlung nach der zweiten Messmethode bestimmt wird,
**dadurch gekennzeichnet, dass**
die Rechen- und Auswerteinheit (16) derart konfiguriert ist, dass die Hämolyse oder der den Einfluss der Hämolyse auf die Messung des Hämatokrits korrigierende Korrekturfaktor auf der Grundlage des nach der ersten und zweiten Messmethode ermittelten Wertes für den Hämatokrit bestimmt wird, wobei die Rechen- und Auswerteinheit (16) derart konfiguriert ist, dass die Hämolyse oder der den Einfluss der Hämolyse auf die Messung des Hämatokrits korrigierende Korrekturfaktor auf der Grundlage der Differenz des nach der ersten und zweiten Messmethode ermittelten Wertes für den Hämatokrit bestimmt wird, wobei die Sende- und Empfangseinheit (14) derart ausgebildet ist, dass nach der ersten Messmethode die in dem Vollblut gestreute Streustrahlung gemessen wird und nach der zweiten Messmethode die durch das Vollblut hindurchtretende Transmissionsstrahlung gemessen wird, die in der Richtung der auf das Vollblut gerichteten Strahlung gerichtet ist.

5. Vorrichtung nach Anspruch 4, **dadurch gekennzeichnet, dass** die Rechen- und Auswerteinheit (16) derart konfiguriert ist, dass die Hämolyserate auf der Grundlage eines vorgegebenen linearen Zusammenhangs zwischen der Differenz des nach der ersten und zweiten Messmethode ermittelten Wertes für den Hämatokrit und der Hämolyse bestimmt wird.

6. Vorrichtung nach Anspruch 4 oder 5, **dadurch gekennzeichnet**, die Sende- und Empfangseinheit (14) derart ausgebildet ist, dass nach der ersten Messmethode die rückwärtsgestreute Streustrahlung gemessen wird, die der in Richtung der auf das Vollblut gerichteten Strahlung entgegen gerichtet ist.

7. Vorrichtung nach einem der Ansprüche 4 bis 6, **dadurch gekennzeichnet** die Vorrichtung eine Einheit (13) mit einem Aufnahmeraum (13A) zur klemmenden Aufnahme einer transparenten Schlauchleitung (6) für das Vollblut oder eine Einheit (13) zur Befestigung einer einen Kanal (19) für das Vollblut aufweisenden Kassette (18) aufweist.

8. Vorrichtung nach einem der Ansprüche 4 bis 7, **dadurch gekennzeichnet, dass** die Sende- und Empfangseinheit (14) aufweist:
einen auf der einen Seite der Schlauchleitung (6) oder des Kanals (19) der Kassette (18) angeordneten Sender (S) zum Bestrahlen der Schlauchleitung oder des Kanals der Kassette mit einer Strahlung in Richtung einer senkrecht zu der Längsachse der Schlauchleitung (6) oder des Kanals (19) verlaufenden Achse,
einen auf der gleichen Seite wie der Sender (S) angeordneten ersten Empfänger (RS) zum Empfangen einer Streustrahlung in Richtung einer Messachse, die senkrecht zu der Längsachse der Schlauchleitung oder des Kanals verläuft und in Längsrichtung der Schlauchleitung oder des Kanals zu der Achse des Sendes im Abstand (x) angeordnet ist, und
einen auf der anderen Seite der Schlauchleitung (6) oder des Kanals (19) angeordneten zweiten Empfänger (TS) zum Empfangen einer Transmissionsstrahlung in Richtung einer Achse, die auf der Achse der auf die Schlauchleitung (6) oder des Kanals (19) gerichteten Strahlung des Senders (S) liegt.

9. Vorrichtung nach einem der Ansprüche 4 bis 7, **dadurch gekennzeichnet**, die Sende- und Empfangseinheit (14) aufweist:
einen auf der einen Seite der Schlauchleitung (6) oder des Kanals (19) der Kassette (18) angeordneten ersten Sender (S1) zum Bestrahlen der Schlauchleitung oder des Kanals der Kassette mit einer Strahlung in Richtung einer senkrecht zu der Längsachse der Schlauchleitung (6) oder des Kanals (19) verlaufenden Achse,
einen auf der anderen Seite der Schlauchleitung (6) oder des Kanals (19) angeordneten zweiten Sender (S2) zum Bestrahlen der Schlauchleitung oder des Kanals mit einer Strahlung in Richtung einer senkrecht zu der Längsachse der Schlauchleitung (6) oder des Kanals (19) verlaufenden Achse, wobei die Achse des zweiten Senders (S2) in Längsrichtung der Schlauchleitung (6) oder des Kanals (19) zu der Achse des ersten Senders im Abstand (x) angeordnet ist, und
einen auf der gleichen Seite wie der zweite Sender (S2) angeordneten Empfänger (TS, RS) zum Empfangen einer rückwärtsgestreuten Strahlung von dem zweiten Sender (S2) und einer Transmissionsstrahlung von dem ersten Sender (S1), wobei die Achse des Empfängers (TS, RS) auf der Achse des ersten Senders (S1) liegt.

10. Verfahren bzw. Vorrichtung nach einem der Ansprüche 1 bis 9, **dadurch gekennzeichnet, dass** die Strahlung Licht mit einer Wellenlänge in einem nicht sichtbaren Bereich von 380 nm bis 780 nm ist.

11. Extrakorporale Blutbehandlungsvorrichtung mit einem extrakorporalen Blutkreislauf (I), der eine Blutzuführleitung (5), die zu einer ersten Kammer (3) einer von einer semipermeablen Membran (2) in die erste Kammer (3) und eine zweite Kammer (4) getrennten Austauscheinheit (1) führt, und eine Blutrückführleitung (6) aufweist, die von der ersten Kammer (3) der Austauscheinheit (1) abgeht, **dadurch gekennzeichnet, dass** die extrakorporale Blutbehandlungsvorrichtung eine Vorrichtung nach einem der Ansprüche 6 bis 10 aufweist.

## Claims

1. A method for measuring hemolysis in whole blood or determining a correction factor for correcting the influence of hemolysis on a hematocrit measurement using the following method steps:
Irradiating the whole blood with a radiation that is directed on the whole blood,
Detecting an emerging radiation with regard to the incoming radiation from a first direction for taking a first measurement according to a first measuring method, and
Detecting an emerging radiation with regard to the incoming radiation from a second direction for taking a second measurement according to a second measuring method,
Determining a first hematocrit value based on the intensity of the radiation emerging from the first direction according to the first measuring method,
Determining a second value for the hematocrit based on the intensity of the radiation emerging from the second direction according to the second measurement,
**characterized in that**
the hemolysis or the correction factor for correcting the influence of the hemolysis on the hematocrit measurement is established on the basis of the value for hematocrit that was determined according to the first and second measuring methods, the hemolysis or the correction factor for correcting the influence of the hemolysis on the hematocrit measurement is determined on the basis of the difference of the hematocrit values determined according to the first and second measuring methods, the radiation emerging from the first direction being the scattered radiation that was scattered in the whole blood and the radiation emerging from the second direction being the transmitted radiation that passes through the whole blood and that is directed in the same direction as the radiation that is directed onto the whole blood.

2. The method according to claim 1, **characterized in that** the hemolysis rate is determined on the basis of a preset linear relationship between the difference of the hematocrit value and the hemolysis rate detected according to the first and second measuring methods.

3. The method according to claim 1 or 2, **characterized in that** the radiation emerging from the first direction is the backscattered radiation that is directed in the opposite direction of the direction of the radiation that is directed onto the whole blood.

4. An apparatus for measuring hemolysis in whole blood or for determining a correction factor that corrects the influence of hemolysis on a hematocrit measurement, with
a transmitting and receiving unit (14) for irradiating the whole blood with a radiation that is directed onto the whole blood and for detecting a radiation that emerges in relation to the incoming radiation from a first direction for taking a first measurement according to a first measuring method and for detecting a radiation that emerges in relation to the incoming radiation from a second direction for taking a second measurement according to a second measuring method,
a computing and analyzer unit (16) that is configured such that a first hematocrit value is determined based on the intensity of the radiation emerging from the first direction according to a first measuring method, and a second hematocrit value is determined based on the intensity of the radiation emerging from the second direction according to a second measuring method,
**characterized in that**
the computing and analyzer unit (16) is configured such that the hemolysis or the correction factor for measuring the influence of the hemolysis on the hematocrit measurement is established on the basis of the hematocrit values detected according to the first and the second measuring methods, the computing and analyzer unit (16) being configured such that the hemolysis or the correction factor for correcting the influence of the hemolysis on the hematocrit measurement is determined on the basis of the difference of the hematocrit values as detected according to the first and second measuring methods, the transmitter and receiver unit (14) being configured such that the scattered radiation that is scattered in the whole blood is measured according to the first measuring method and the transmitter and receiver unit (14) being configured such that the transmitted radiation that passes through the whole blood is measured according to the second measuring method and that is directed in the direction of the radiation that is directed onto the whole blood.

5. The apparatus according to claim 4, **characterized in that** the computing and analyzer unit (16) is configured such that the hemolysis rate is determined on the basis of a preset linear relationship between the difference of the hematocrit values and the hemolysis as detected according to the first and the second measuring methods.

6. The apparatus according to claim 4 or 5, **characterized in that** the transmitter and receiver unit (14) is configured such that the backscattered scattered radiation is measured according to the first measuring method and that is directed opposite relative to the direction of the radiation that is directed in the direction of the whole blood.

7. The apparatus according to any one of the claims 4 to 6, **characterized in that** the apparatus includes a unit (13) with a receptacle (13A) for the clamped-like receiving of a transparent hose line (6) for the whole blood or a unit (13) for mounting a cartridge (18) that includes a channel (19) for the whole blood.

8. The apparatus according to any one of the claims 4 to 7, **characterized in that** the transmitter and receiver unit (14) includes:
a transmitter (S) that is disposed on the one side of the hose line (6) or the channel (19) of the cartridge (18) for irradiating the hose line or the channel of the cartridge with a radiation in the direction of an axis that runs vertically relative to the longitudinal axis of the hose line (6) or the channel (19),
a first receiver (RS) that is disposed on the same side as the transmitter (S) for receiving a scattered radiation in the direction of a measurement axis that runs vertically relative to the longitudinal axis of the hose line or the channel and that is disposed in the longitudinal direction of the hose line or the channel observing a spacing (x) relative to the transmitter, and
a second receiver (TS) that is disposed on the other side of the hose line (6) or the channel (19) for receiving transmitted radiation in the direction of an axis that is located on the axis of the radiation of the transmitter (S) which is directed to the hose line (6) or the channel (19).

9. The apparatus according to any one of the claims 4 to 7, **characterized in that** the transmitter and receiver unit (14) includes:
a first transmitter (S1) that is disposed on the one side of the hose line (6) or the channel (19) of the cartridge (18) for irradiating the hose line or the channel of the cartridge with a radiation in the direction of an axis running vertically in the direction of the longitudinal axis of the hose line (6) or the channel (19),
a second transmitter (S2) that is disposed on the other side of the hose line (6) or the channel (19) for irradiating the hose line or the channel with a radiation in the direction of an axis running vertically in relation to the longitudinal axis of the hose line (6) of the channel (19), wherein the axis of the second transmitter (S2) is disposed observing a spacing (x) relative to the axis of the first transmitter in the longitudinal direction of the hose line (6) or the channel (19), and
a receiver (TS, RS) that is disposed on the same side as the second transmitter (S2) for receiving a backscattered radiation from the second transmitter (S2) and a transmitted radiation from the first transmitter (S1), wherein the axis of the receiver (TS, RS) lies on the axis of the first transmitter (S1).

10. The method and/or the apparatus according to any one of the claims 1 to 9, **characterized in that** the radiation is light in the visible range of between 380 nm and 780 nm.

11. An extracorporeal blood treatment apparatus with an extracorporeal blood circulation (I) that comprises a blood supply line (5) leading to a first chamber of an exchange unit (1), which is divided by a semi-permeable membrane (2) into the first chamber (3) and a second chamber (4), and comprises a blood return line (6) that branches off from the first chamber (3) of the exchange unit (1), **characterized in that** the extracorporeal blood treatment apparatus comprises a device according to any one of the claims 6 to 10.

## Revendications

1. Procédé pour la mesure de l'hémolyse dans le sang total ou la détermination d'un facteur de correction corrigeant l'influence de l'hémolyse sur une mesure de l'hématocrite avec les étapes de procédé suivantes:
irradiation du sang total avec un rayonnement optique dirigé vers le sang total,
détection d'un rayonnement émergeant d'une première direction par rapport au rayonnement incident pour effectuer une première mesure selon une première méthode de mesure et
détection d'un rayonnement émergeant d'une seconde direction par rapport au rayonnement incident pour effectuer une seconde mesure selon une seconde méthode de mesure,
détermination d'une première valeur pour l'hématocrite à partir de l'intensité du rayonnement émergeant de la première direction selon la première méthode de mesure,
détermination d'une seconde valeur pour l'hématocrite à partir de l'intensité du rayonnement émergeant de la seconde direction selon la seconde méthode de mesure,
**caractérisé en ce que**
l'hémolyse ou le facteur de correction corrigeant l'influence de l'hémolyse sur la mesure de l'hématocrite est déterminé sur la base de la valeur pour l'hématocrite déterminée selon les première et seconde méthodes de mesure, où l'hémolyse ou le facteur de correction corrigeant l'influence de l'hémolyse sur la mesure de l'hématocrite est déterminé sur la base de la différence de la valeur pour l'hématocrite déterminée selon la première et la seconde méthode de mesure, où le rayonnement émergeant de la première direction est le rayonnement diffusé diffusé dans le sang total et le rayonnement émergeant de la seconde direction est le rayonnement de transmission traversant le sang total qui est dirigé dans la direction du rayonnement dirigé vers le sang total.

2. Procédé selon la revendication 1, **caractérisé en ce que** le taux d'hémolyse est déterminé sur la base d'une relation linéaire prédéterminée entre la différence de la valeur pour l'hématocrite et le taux d'hémolyse déterminée selon la première et la seconde méthode de mesure.

3. Procédé selon la revendication 1 ou 2, **caractérisé en ce que** le rayonnement émergeant de la première direction est le rayonnement diffusé diffusé vers l'arrière qui est dirigé à l'opposé de la direction du rayonnement dirigé vers le sang total.

4. Dispositif pour la mesure de l'hémolyse dans le sang total ou la détermination d'un facteur de correction corrigeant l'influence de l'hémolyse sur une mesure de l'hématocrite avec
une unité d'émission et de réception (14) pour irradier le sang total avec un rayonnement optique dirigé vers le sang total et pour détecter un rayonnement émergeant d'une première direction par rapport au rayonnement incident pour effectuer une première mesure selon une première méthode de mesure et pour détecter un rayonnement émergeant d'une seconde direction par rapport au rayonnement incident pour effectuer une seconde mesure selon une seconde méthode de mesure,
une unité de calcul et d'évaluation (16) qui est configurée de telle sorte qu'une première valeur pour l'hématocrite est déterminée à partir de l'intensité du rayonnement émergeant de la première direction selon la première méthode de mesure et une seconde valeur pour l'hématocrite est déterminée à partir de l'intensité du rayonnement émergeant de la seconde direction selon la seconde méthode de mesure,
**caractérisé en ce que**
l'unité de calcul et d'évaluation (16) est configurée de telle sorte que l'hémolyse ou le facteur de correction corrigeant l'influence de l'hémolyse sur la mesure de l'hématocrite est déterminé sur la base de la valeur pour l'hématocrite déterminée selon la première et la seconde méthode de mesure, où l'unité de calcul et d'évaluation (16) est configurée de telle sorte que l'hémolyse ou le facteur de correction corrigeant l'influence de l'hémolyse sur la mesure de l'hématocrite est déterminé sur la base de la différence de la valeur pour l'hématocrite déterminée selon la première et la seconde méthode de mesure, où l'unité d'émission et de réception (14) est conçue de telle sorte que le rayonnement diffusé diffusé dans le sang total est mesuré selon la première méthode de mesure et le rayonnement de transmission traversant le sang total qui est dirigé dans la direction du rayonnement dirigé vers le sang total est mesuré selon la seconde méthode de mesure.

5. Dispositif selon la revendication 4, **caractérisé en ce que** l'unité de calcul et d'évaluation (16) est configurée de telle sorte que le taux d'hémolyse est déterminé sur la base d'une relation linéaire prédéterminée entre la différence de la valeur pour l'hématocrite et l'hémolyse déterminée selon la première et la seconde méthode de mesure.

6. Dispositif selon la revendication 4 ou 5, **caractérisé en ce que** l'unité d'émission et de réception (14) est conçue de telle sorte que le rayonnement diffusé diffusé vers l'arrière qui est dirigé à l'opposé de la direction du rayonnement dirigé vers le sang total est mesuré selon la première méthode de mesure.

7. Dispositif selon l'une des revendications 4 à 6, **caractérisé en ce que** le dispositif présente une unité (13) avec un espace de réception (13A) pour la réception avec serrage d'une conduite flexible transparente (6) pour le sang total ou une unité (13) pour la fixation d'une cassette (18) présentant un canal (19) pour le sang total.

8. Dispositif selon l'une des revendications 4 à 7, **caractérisé en ce que** l'unité d'émission et de réception (14) présente:
un émetteur (S) agencé d'un côté de la conduite flexible (6) ou du canal (19) de la cassette (18) pour irradier la conduite flexible ou le canal de la cassette avec un rayonnement dans la direction d'un axe s'étendant perpendiculairement à l'axe longitudinal de la conduite flexible (6) ou du canal (19),
un premier récepteur (RS) agencé du même côté que l'émetteur (S) pour recevoir un rayonnement diffusé dans la direction d'un axe de mesure qui s'étend perpendiculairement à l'axe longitudinal de la conduite flexible ou du canal et disposé dans la direction longitudinale de la conduite flexible ou du canal à une distance (x) par rapport à l'axe de l'émetteur, et
un second récepteur (TS) agencé de l'autre côté de la conduite flexible (6) ou du canal (19) pour recevoir un rayonnement de transmission dans la direction d'un axe qui est situé sur l'axe du rayonnement de l'émetteur (S) dirigé vers la conduite flexible (6) ou le canal (19).

9. Dispositif selon l'une des revendications 4 à 7, **caractérisé en ce que** l'unité d'émission et de réception (14) présente:
un premier émetteur (S1) disposé d'un côté de la conduite flexible (6) ou du canal (19) de la cassette (18) pour irradier la conduite flexible ou le canal de la cassette avec un rayonnement dans la direction d'un axe s'étendant perpendiculairement à l'axe longitudinal de la conduite flexible (6) ou du canal (19),
un second émetteur (S2) agencé de l'autre côté de la conduite flexible (6) ou du canal (19) pour irradier la conduite flexible ou le canal avec un rayonnement dans la direction d'un axe s'étendant perpendiculairement à l'axe longitudinal de la conduite flexible (6) ou du canal (19), où l'axe du second émetteur (S2) est disposé dans la direction longitudinale de la conduite flexible (6) ou du canal (19) à une distance (x) par rapport à l'axe du premier émetteur, et
un récepteur (TS, RS) disposé du même côté que le second émetteur (S2) pour recevoir un rayonnement diffusé vers l'arrière du second émetteur (S2) et un rayonnement de transmission du premier émetteur (S1), où l'axe du récepteur (TS, RS) est situé sur l'axe du premier émetteur (S1).

10. Procédé ou dispositif selon l'une des revendications 1 à 9, **caractérisé en ce que** le rayonnement est de la lumière avec une longueur d'onde dans un domaine non visible de 380 nm à 780 nm.

11. Dispositif de traitement du sang extracorporel avec un circuit sanguin extracorporel (I), qui présente une conduite d'amenée du sang (5), qui conduit à une première chambre (3) d'une unité d'échange (1) divisée par une membrane semi-perméable (2) en la première chambre (3) et une seconde chambre (4), et une conduite de retour du sang (6) qui part de la première chambre (3) de l'unité d'échange (1), **caractérisé en ce que** le dispositif de traitement du sang extracorporel présente un dispositif selon l'une des revendications 6 à 10.
